# EUROPEAN PATENT SPECIFICATION

(11) **EP 2 080 530 B1**
(45) Date of publication and mention of the grant of the patent: **18.08.2010**
(21) Application number: 09150196.5
(22) Date of filing: 08.01.2009
(51) Int. Cl.: A61M 1/00

(54) **Chest drainage device**
Thoraxdrainagevorrichtung
Dispositif de drainage thoracique

(30) Priority: 18.01.2008 IT MI20080076
(43) Date of publication of application: 22.07.2009
(73) Proprietor: Eurosets S.r.l., 41036 Medolla (MO) (IT)
(72) Inventor: Fontanili, Paolo, 42015, CORREGGIO RE (IT); Paratelli, Ruggero, 41036, MEDOLLA MO (IT); Balugani, Fabio, 41032, CAVEZZO MO (IT)
(74) Representative: Modiano, Micaela Nadia

(56) References cited:
- GB-A- 2 166 055
- US-A- 3 847 152
- US-A- 4 210 174
- US-A1- 2004 055 639

## Description

The present invention relates to a chest drainage device.

It is known that there are devices designed to be connected to the chest cavity of a patient after a surgical procedure or after a trauma by means of a tube that conveys to the device the exuded liquids and the air losses that exit from the cavity by way of the suction determined by the vacuum created within the device itself, or by gravity; such devices are known as "chest drainage" devices.

Known devices are divided into interconnected contiguous chambers.

A first chamber is designed to collect the exuded liquids that originate, together with the air losses, from the patient and accumulate on the bottom of the chamber, and is followed by at least one second chamber, known as sealing chamber, which is designed to receive the air losses that originate from the first chamber, preventing their return thanks to the presence of a one-way valve, or of a layer of water that is present on the bottom, at which it is possible to provide a device for measuring the air losses.

A connector adapted to be connected to a source of suction to provide the vacuum required inside the device is provided at said second sealing chamber, or at a third chamber that is adjacent thereto.

Such suction source is constituted usually by a vacuum line of a general system, which is notoriously subject to fluctuations, and therefore it becomes necessary to provide the devices described above with a vacuum adjustment device, such as to ensure absolute constancy of the vacuum in order to ensure correct operation.

Vacuum adjustment devices are known in the background art according to different embodiments, and one of them is disclosed in US 6,959,722 B2 by the same Applicant.

Such embodiment comprises a valve that has an air passage duct that is connected to the atmosphere and comprises a flow control element that is adapted to be attracted into a closure position, in abutment against a sealing surface, by the action of a permanent magnet, which is supported by means that are associated in an adjustable position with the body of the valve and can move along the axis of such valve. It is thus possible to achieve arrangements of such magnet at differentiated distances from the sealing surface, thus converting the attraction force applied by the magnet to the flow control element, which is inversely proportional to their mutual distance, with the consequent possibility to vary the degree of vacuum, which is indeed proportional to such force.

In Italian Patent 1322324 the same Applicant has disclosed an embodiment of the vacuum adjustment means that is similar to the one described above but in which the permanent magnet is associated with the valve body in a fixed position.

In the above embodiments, the axis of the valve of the vacuum adjustment device is perpendicular to the resting surface that lies at the base of the chest drainage device to which the valve is applied, and is thus designed to assume a vertical arrangement in the normal conditions of use of the drainage device.

This embodiment provides excellent results, but improvements are still needed.

The aim of the present invention is to obtain such improvements that are particularly oriented toward ensuring optimum conditions of stability of the flow control element of the valve.

Another object of the invention is to provide improvements related to the one-way valve that is optionally present in the sealing chamber and to the maximum negative pressure valve that always accompanies the presence of said one-way valve, as described in greater detail hereinafter.

The proposed aim and objects are achieved by a chest drainage device according to the invention, characterized in that it comprises the characteristics according to the appended claims.

Further characteristics and advantages of the present invention will become better apparent from the description of a preferred but not exclusive embodiment thereof, illustrated by way of non-limiting example in the accompanying drawings, wherein:

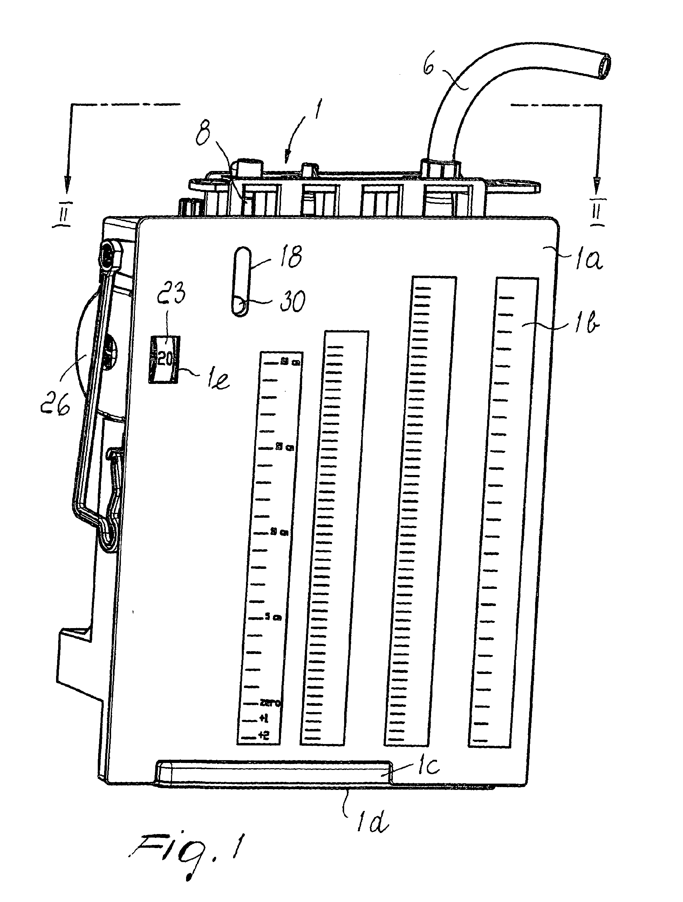

Figure 1 is a perspective view of the chest drainage device according to the invention;

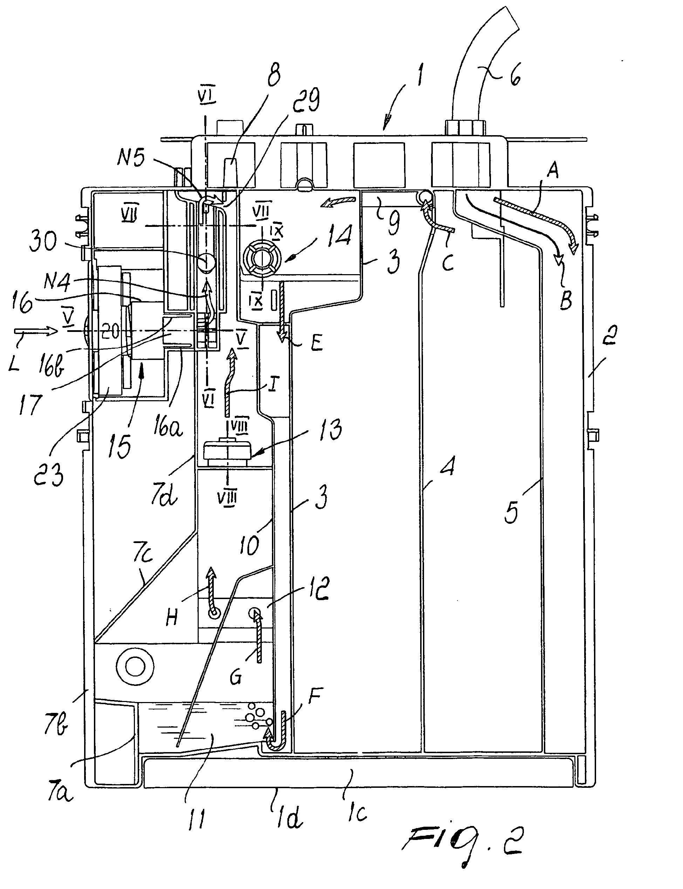

Figure 2 is a sectional view, taken along the line II-II of Figure 1, obtained in practice by removing the outer wall provided with graduated windows that is clearly visible in Figure 1;

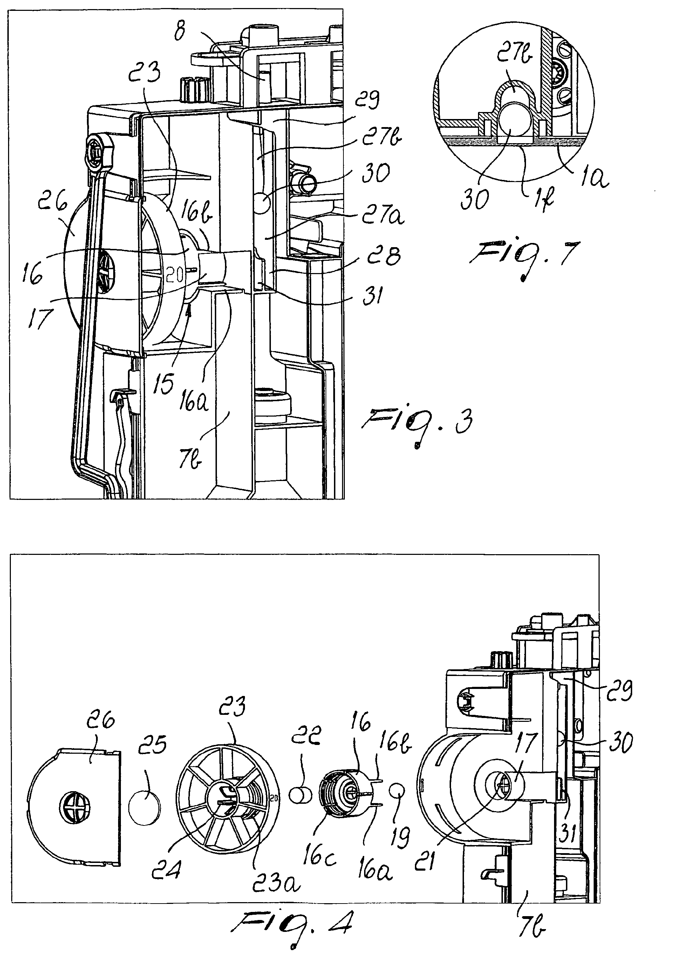

Figures 3 and 4 are respectively a perspective view and an exploded view of a detail of Figure 2;
Figures 5, 6, 7 are sectional views, taken respectively along the lines V-V, VI-VI and VII-VII of Figure 2, and show the presence also of the wall provided with graduated windows, which is actually removed, as mentioned, in said Figure 2;
Figures 8 and 9 are sectional views, taken along the lines VIII-VIII and IX-IX of Figure 2.

With reference to the figures, the reference numeral 1 generally designates the device, which comprises a flat outer wall 1a, provided with transparent regions 1b for viewing the content of the several chambers that are present within the device, which will be described hereafter; the device is provided with a base 1c, which has a resting surface 1d.

The first collection chamber, delimited by walls 2 and 3 and divided into three subchambers by partitions 4 and 5, is designed to be connected by means of a tube 6 to the chest cavity of a patient, so as to receive the air losses along the arrow A and the exuded liquids along the arrow B in order to collect these last at the bottom.

The second sealing chamber is contiguous to the first chamber and is delimited by the wall 3 and by a wall formed by portions 7a, 7b, 7c, 7d; it is designed to be connected by means of a connector 8 to a suction source and is connected by means of a duct 9 to the first chamber, so that the same degree of vacuum that determines the suction of the exuded liquids and of the air losses occurs therein.

Whereas, as mentioned, the exuded liquids collect on the bottom of the first chamber, the air losses, following the arrows C and D, pass through the duct 9 and reach the second chamber, where they are directed by a partition 10, along the arrow E, toward the bottom in order to penetrate by following the arrow F a water layer 11 that acts as a seal for such losses, preventing their return, and constitutes an integral part of a device for measuring the losses, which comprises also a cock 12. In output from the device along the arrows G, H, which show the crossing of the cock 12, the air losses arrive along the arrow I to the connector 8 to be evacuated.

Within the second sealing chamber there is a one-way valve, generally designated by the reference numeral 13, which will be discussed in greater detail hereinafter, which is a further element in addition to the water layer 11 in preventing the return of the air losses to the first chamber.

Such valve 13, whose presence evidently relieves the operators from providing the water layer 11 if they are not interested in measuring air losses, might also be omitted, and in this case the presence of the water layer becomes indispensable.

The valve 13, when provided, requires in a known manner, as necessary, the presence in the first chamber of a maximum negative pressure valve, generally designated by the reference numeral 14, which also will be described hereinafter; it is designed to prevent the degree of vacuum that is present in the first chamber from reaching, for example due to coughing, an excessive value.

At the second sealing chamber, and more precisely at the portion of wall 7d that delimits the chamber, there is the vacuum adjustment device, which comprises a valve, generally designated by the reference numeral 15, which is now described in detail.

The valve 15 comprises a body 16, which is jointly connected by adhesive bonding in the presence of references 16a, 16b to an end of a receptacle 17 that is provided monolithically within the structure of the device.

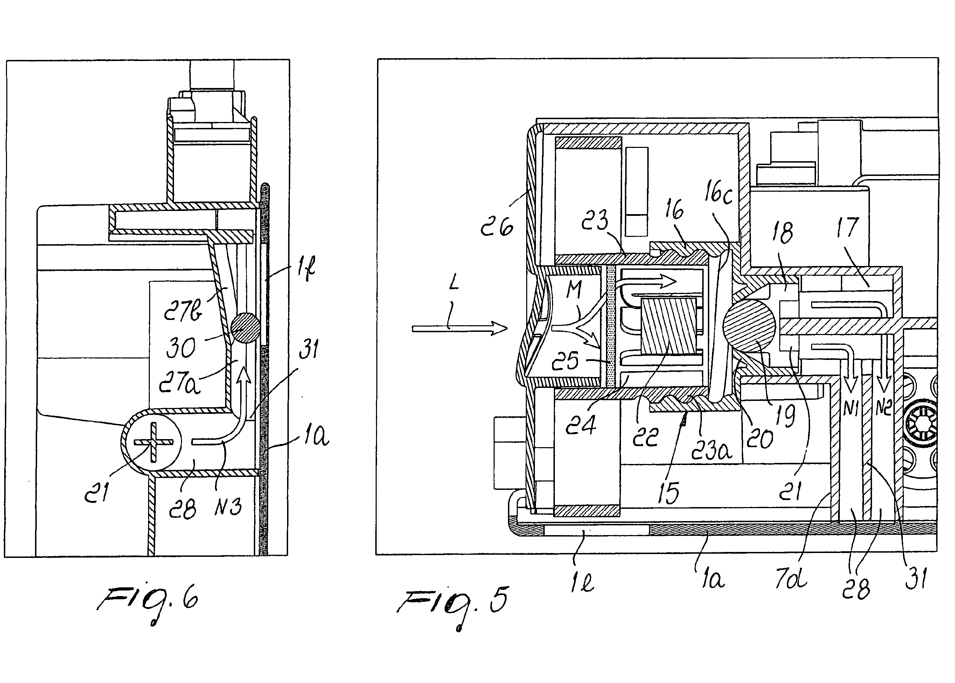

A duct 18 for the passage of the atmospheric air that is present in the body 16 accommodates a spherical flow control element 19, which is made of magnetic steel and can move between the closure position, shown in Figure 5 in contact with the sealing surface provided at the end of a frustum-shaped terminal 20 that is jointly connected to the body 16, and a fully open position in contact with an abutment 21 having a cross-shaped cross-section, provided within the receptacle 17.

A permanent magnet 22 that attracts the flow control element 19 toward the closure position is supported by a ring 23, which is associated in an adjustable position with the body 16 of the valve by means of a thread 23a, which is shaped complementarily with respect to a thread 16c of the body and is provided with fins 24 that support the magnet 22, allowing the passage of atmospheric air, which has entered along the arrow L, following the arrow M after passing through a felt 25; on the surface of the ring 23, which is protected by a cover 26, there are numeric indications, such as the numeral 20 that appears in the figures, which are suitable to appear selectively through an opening le formed in the wall la of the device in order to provide an indication of the degree of vacuum provided by the device.

According to an important characteristic of the invention, the axis of the valve 15 is contained on a plane that is parallel to the resting surface 1d of the device and has a direction that is parallel to the outer wall 1a of the device, thus achieving optimum conditions of stability of the flow control element 19 for any vacuum condition.

Similar conditions are obtained in a variation of the invention in which the axis of the valve 15 is again contained on a plane that is parallel to the resting surface 1d, but with a direction that is perpendicular to the outer wall 1a of the device.

It should be noted immediately that all the remarks made above and referred to a valve in which the permanent magnet is supported in an adjustable position apply identically if the valve comprised in the vacuum adjustment device comprises a permanent magnet associated with the body of the valve in a fixed position.

A further characteristic of the invention consists in that the diameter of the flow control element 19 and of the duct 18 for accommodating it have a numeric ratio of no more than 0.7.

The receptacle 17 is connected to the second chamber for sealing the device with the interposition of means for indicating the flow of atmospheric air along the arrows N1, N2, N3, N4, N5, the passage being of course allowed when the flow control element 19 abandons the closure position shown in Figure 5.

The indication means comprise the duct shaped according to a first portion 27a having a constant cross-section and a second portion 27b having a cross-section that increases in the direction of motion of the air, which is derived from the receptacle 17 by means of a portion 28 and is open at a top 29 into said second sealing chamber.

The duct accommodates a ball 30, which is made of plastics and is adapted to be conveyed by the air that passes through the valve from a position that rests on a retention element 31 at the base of the duct, assumed in the absence of the air, until it faces an opening 1f, which is elongated in the direction of the duct at the portion 27b and is closed by means of transparent material, so that it is visible by an operator so as to indicate to him indeed the passage of air.

More specifically, the ball 30 is at the base of the window 1f, i.e., in the position shown in the figures, in the presence of the minimal flow of atmospheric air that enters the device adapted to ensure the value of the set degree of vacuum; in the case of greater air inflows, the ball 30 is in a higher position, and the breadth of the air passage section at the portion 27b of the duct is such as to avoid unwanted throttlings of the air.

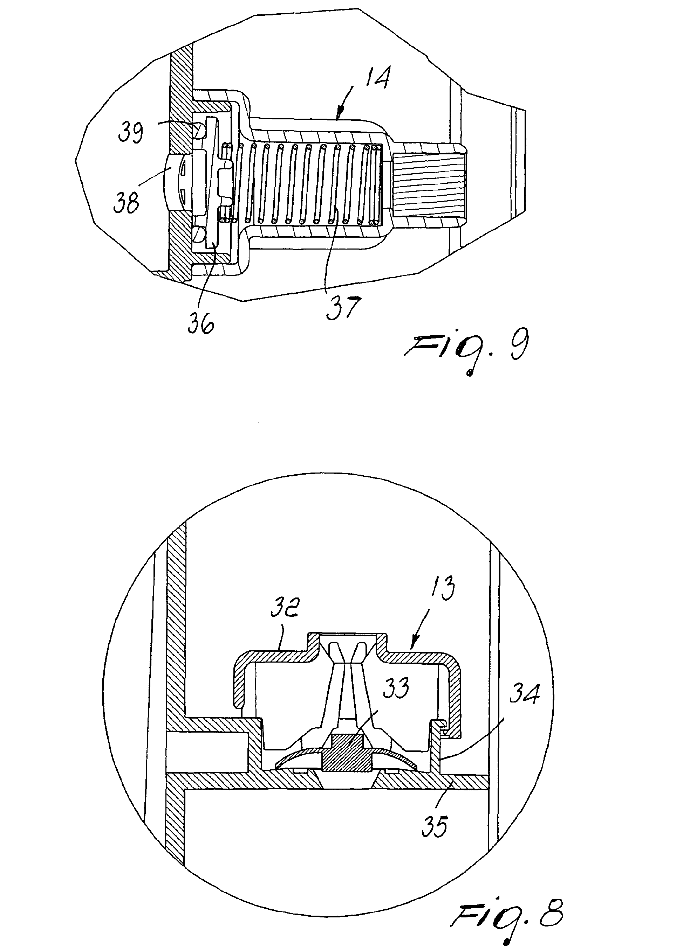

Considering now, with reference to Figure 8, the one-way valve 13, it is noted that the containment body 32 of the flow control element 33 is engaged with the base 34, which is formed monolithically with the structure 35 of the device.

Finally, Figure 9 illustrates the maximum negative pressure valve 14, which comprises a flow control element 36 pushed by a spring 37 to block a port 38 for connection to the atmosphere and provided with an O-Ring gasket 39 designed to come into contact with the flat wall at the rim of the port.

The described invention is susceptible of numerous modifications and variations in addition to the ones described above, all of which are within the scope of the appended claims; all the details may further be replaced with other technically equivalent elements.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A chest drainage device (3), comprising contiguous interconnected chambers which are adapted to be placed in partial vacuum by being connected to a suction source capable of generating, within the device, such a vacuum as to convey from a patient the exuded liquids and the air losses, said device being provided with a vacuum adjustment device that comprises a valve (15) provided with a duct (18) for the passage of air which is connected to the atmosphere and comprises a flow control element (19) adapted to be attracted into the closure position in abutment against a sealing surface by the action of a permanent magnet (22), **characterized in that** the axis of the valve is contained on a plane that is parallel to the resting surface (1d) of the device that lies at the base of said device.

2. The device according to claim 1, comprising an outer wall provided with transparent regions for viewing the content of the several contiguous chambers, **characterized in that** the axis of the valve is contained on a plane that is parallel to the resting surface of the device and has a direction that is parallel to said wall.

3. The device according to claim 1, comprising an outer wall provided with transparent regions for viewing the content of the several contiguous chambers, **characterized in that** the axis of the valve is contained on a plane that is parallel to the resting surface of the device and has a direction that is perpendicular to said wall.

4. The device according to one or more of the preceding claims, **characterized in that** the valve body is jointly connected, in the presence of precise references, to an end of a receptacle that is provided monolithically within the structure of the device and is connected at the other end, with the interposition of means for indicating the flow of atmospheric air, to the chambers in partial vacuum that are present in the device, the spherical flow control element being movable within the air passage duct that is present in the body of the valve between a closure position, in contact with a sealing surface formed at the end of a frustum-shaped terminal that is jointly connected to said body, and a fully open position in contact with an abutment element formed in said receptacle, the permanent magnet being supported by means that comprise a ring that is associated with the valve body by means of a thread and is provided with fins that support the magnet, allowing the passage of air.

5. The device according to one or more of the preceding claims, **characterized in that** the ratio between the diameters of the spherical flow control element and of the air passage duct that contains said flow control element has a value of no more than 0.7.

6. The device according to one or more of the preceding claims, comprising means for indicating the passage of atmospheric air through the valve comprised within the vacuum adjustment device, **characterized in that** said means for indicating the passage of atmospheric air through the valve comprise a duct that extends from the receptacle of the valve body provided in the structure of the device and is connected to the chambers in partial vacuum that are present within said device, said duct accommodating a body that is adapted to be conveyed by the air that passes through the valve from a position proximate to the base of said duct, assumed in the absence of air, until it faces a window that is elongated in the direction of the duct and is closed by means of transparent material, so as to be visible from the outside, said duct being shaped according to a first portion that has constant cross-section and extends from the base to the lower end of the window, and a second portion whose cross-section increases in the direction of motion of the air and is arranged at said window.

7. The device according to one or more of the preceding claims, comprising, within the sealing chamber, a one-way valve that is adapted to allow the passage of the air losses that originate from the chamber for collecting the exuded liquids toward the suction source, preventing their return, said valve comprising a body for containing a flow control element and a base for engaging said body, **characterized in that** said base is formed monolithically with the structure of the device.

8. The device according to one or more of the preceding claims, comprising, within the chamber for collecting the exuded liquids, a maximum negative pressure valve, which comprises a flow control element that is pushed by an appropriately calibrated spring so as to block a port for connection to the atmosphere, **characterized in that** said flow control element is provided with an O-ring gasket designed to come into contact with the flat wall at the rim of said port.

## Patentansprüche

1. Thoraxdrainagevorrichtung (1), umfassend miteinander verbundene benachbarte Kammern, die dazu geeignet sind, in Teilvakuum versetzt zu werden, indem sie mit einer Saugquelle verbunden werden, die in der Lage ist, in der Vorrichtung ein derartiges Vakuum zu erzeugen, dass die abgesonderten Flüssigkeiten und die Luftverluste von einem Patienten abgeleitet werden, wobei die Vorrichtung mit einer Vakuumeinstellvorrichtung versehen ist, die ein Ventil (15) umfasst, das mit einem Kanal (18) für den Durchgang von Luft versehen ist, der mit der Außenumgebung verbunden ist und ein Strömungsregelelement (19) umfasst, das dazu geeignet ist, durch die Wirkung eines Permanentmagneten (22) in die Schließstellung in Anlage gegen eine Dichtfläche angezogen zu werden, **dadurch gekennzeichnet, dass** die Achse des Ventils auf einer Ebene enthalten ist, die parallel zu der Standfläche (1d) der Vorrichtung ist, die an der Basis der Vorrichtung liegt.

2. Vorrichtung nach Anspruch 1, umfassend eine äußere Wand, die mit transparenten Bereichen zum Betrachten des Inhalts der verschiedenen benachbarten Kammern versehen ist, **dadurch gekennzeichnet, dass** die Achse des Ventils auf einer Ebene enthalten ist, die parallel zu der Standfläche der Vorrichtung ist und eine Richtung hat, die parallel zu der Wand ist.

3. Vorrichtung nach Anspruch 1, umfassend eine äußere Wand, die mit transparenten Bereichen zum Betrachten des Inhalts der verschiedenen benachbarten Kammern versehen ist, **dadurch gekennzeichnet, dass** die Achse des Ventils auf einer Ebene enthalten ist, die parallel zu der Standfläche der Vorrichtung ist und eine Richtung hat, die senkrecht zu der Wand ist.

4. Vorrichtung nach einem oder mehreren der vorhergehenden Ansprüche, **dadurch gekennzeichnet, dass** der Ventilkörper in Gegenwart von präzisen Referenzen mit einem Ende einer Aufnahme, die monolithisch in der Struktur der Vorrichtung vorgesehen ist, zusammenwirkend verbunden ist, und mit dem anderen Ende mit den in der Vorrichtung vorhandenen in Teilvakuum befindlichen Kammern verbunden ist, wobei Mittel zum Anzeigen der Strömung von Außenluft dazwischen angeordnet sind, wobei das kugelförmige Strömungsregelelement in dem in dem Körper des Ventils vorhandenen Luftdurchgangskanal bewegbar ist zwischen einer Schließstellung in Kontakt mit einer Dichtfläche, die am Ende eines kegelstumpfförmigen Anschlusses ausgebildet ist, der mit dem Körper zusammenwirkend verbunden ist, und einer vollkommen offenen Stellung in Kontakt mit einem Anschlagelement, das in der Aufnahme ausgebildet ist, wobei der Permanentmagnet von Mitteln gehalten ist, die einen Ring umfassen, der mit dem Ventilkörper mittels eines Gewindes verbunden ist und mit Rippen versehen ist, die den Magneten halten und dabei den Durchgang von Luft erlauben.

5. Vorrichtung nach einem oder mehreren der vorhergehenden Ansprüche, **dadurch gekennzeichnet, dass** das Verhältnis zwischen den Durchmessern des kugelförmigen Strömungsregelelements und des das Strömungsregelelement enthaltenden Luftdurchgangskanals einen Wert von nicht mehr als 0,7 hat.

6. Vorrichtung nach einem oder mehreren der vorhergehenden Ansprüche, umfassend Mittel zum Anzeigen des Durchgangs von Außenluft durch das in der Vakuumeinstellvorrichtung enthaltene Ventil, **dadurch gekennzeichnet, dass** die Mittel zum Anzeigen des Durchgangs von Außenluft durch das Ventil einen Kanal umfassen, der von der in der Struktur der Vorrichtung vorgesehenen Aufnahme des Ventilkörpers ausgeht und mit den in Teilvakuum befindlichen Kammern verbunden ist, die in der Vorrichtung vorhanden sind, wobei der Kanal einen Körper beherbergt, der dazu geeignet ist, von der durch das Ventil strömenden Luft von einer Position nahe der Basis des Kanals, die in Abwesenheit von Luft eingenommen wird, getragen zu werden, bis er einem Fenster gegenüber steht, das in der Richtung des Kanals gestreckt ist und mittels eines transparenten Materials geschlossen ist, so dass er von außen sichtbar ist, wobei der Kanal gemäß einem ersten Abschnitt, der einen konstanten Querschnitt hat und von der Basis zu dem unteren Ende des Fensters verläuft, und einem zweiten Abschnitt geformt ist, dessen Querschnitt in Bewegungsrichtung der Luft zunimmt und an dem Fenster angeordnet ist.

7. Vorrichtung nach einem oder mehreren der vorhergehenden Ansprüche, umfassend ein Einwegventil in der Dichtungskammer, das dazu geeignet ist, den Durchgang der Luftverluste, die von der Kammer zum Sammeln der abgesonderten Flüssigkeiten strammen, zu der Saugquelle zu ermöglichen und ihren Rückfluss verhindert, wobei das Ventil einen Körper zum Beherbergen eines Strömungsregelements und eine Basis zum Berühren des Körpers umfasst, **dadurch gekennzeichnet, dass** die Basis monolithisch mit der Struktur der Vorrichtung ausgebildet ist.

8. Vorrichtung nach einem oder mehreren der vorhergehenden Ansprüche, umfassend ein Maximum-Unterdruckventil in der Kammer zum Sammeln der abgesonderten Flüssigkeiten, das ein Strömungsregelelement umfasst, das von einer geeignet kalibrierten Feder so gedrückt wird, dass es eine Öffnung zur Verbindung mit der Außenumgebung blockiert, **dadurch gekennzeichnet, dass** das Strömungsregelelement mit einer O-Ring-Dichtung ausgestattet ist, die so ausgelegt ist, dass sie mit der flachen Wand an der Kante der Öffnung in Kontakt kommt.

## Revendications

1. Dispositif de drainage thoracique (3) comprenant des chambres interconnectées contiguës qui sont adaptées pour être placées sous vide partiel en étant raccordées à une source d'aspiration capable de générer, à l'intérieur du dispositif, un vide tel qu'il transporte depuis un patient, les liquides exsudés et les pertes d'air, ledit dispositif étant prévu avec un dispositif d'ajustement de vide qui comprend une soupape (15) prévue avec un conduit (18) pour le passage de l'air qui est raccordé à l'atmosphère et comprend un élément de contrôle d'écoulement (19) adapté pour être attiré en position de fermeture en butée contre une surface d'étanchéité par l'action d'un aimant permanent (22), **caractérisé en ce que** l'axe de la soupape est contenu sur un plan qui est parallèle à la surface de repos (1d) du dispositif qui se trouve au niveau de la base dudit dispositif.

2. Dispositif selon la revendication 1, comprenant une paroi externe prévue avec des régions transparentes pour observer le contenu de plusieurs chambres contiguës, **caractérisé en ce que** l'axe de la soupape est contenu sur un plan qui est parallèle à la surface de repos du dispositif et a une direction qui est parallèle à ladite paroi.

3. Dispositif selon la revendication 1, comprenant une paroi externe prévue avec des régions transparentes pour observer le contenu de plusieurs chambres contiguës, **caractérisé en ce que** l'axe de la soupape est contenu sur un plan qui est parallèle à la surface de repos du dispositif et a une direction qui est perpendiculaire à ladite paroi.

4. Dispositif selon une ou plusieurs des revendications précédentes, **caractérisé en ce que** le corps de soupape est raccordé conjointement, en présence de références précises, à une extrémité d'un réceptacle qui est prévu de manière monolithique à l'intérieur de la structure du dispositif et est raccordé au niveau de l'autre extrémité, avec l'interposition de moyens pour indiquer l'écoulement de l'air atmosphérique, jusqu'aux chambres sous vide partiel qui sont présentes dans le dispositif, l'élément de contrôle d'écoulement sphérique étant mobile à l'intérieur du conduit de passage d'air qui est présent dans le corps de la soupape entre une position de fermeture, en contact avec une surface étanche formée au niveau de l'extrémité d'une borne de forme tronconique qui est raccordée conjointement audit corps, et une position complètement ouverte en contact avec un élément de butée formé dans ledit réceptacle, l'aimant permanent étant supporté par des moyens qui comprennent une bague qui est associée au corps de soupape au moyen d'un filetage et est prévue avec des ailettes qui supportent l'aimant, permettant le passage de l'air.

5. Dispositif selon une ou plusieurs des revendications précédentes, **caractérisé en ce que** le rapport entre les diamètres de l'élément de contrôle d'écoulement sphérique et du conduit de passage d'air qui contient ledit élément de contrôle d'écoulement, a une valeur non supérieure à 0,7.

6. Dispositif selon une ou plusieurs des revendications précédentes, comprenant des moyens pour indiquer le passage de l'air atmosphérique à travers la soupape comprise à l'intérieur du dispositif d'ajustement de vide, **caractérisé en ce que** lesdits moyens pour indiquer le passage de l'air atmosphérique à travers la soupape comprennent un conduit qui s'étend à partir du réceptacle du corps de soupape prévu dans la structure du dispositif et est raccordé aux chambres sous vide partiel qui sont présentes à l'intérieur dudit dispositif, ledit conduit logeant un corps qui est adapté pour être transporté par l'air qui passe à travers la soupape à partir d'une position à proximité de la base dudit conduit, prise en l'absence d'air, jusqu'à ce qu'il fasse face à une fenêtre qui est allongée dans la direction du conduit et est fermée au moyen d'un matériau transparent, afin d'être visible depuis l'extérieur, ledit conduit étant formé selon une première partie qui a une section transversale constante et s'étend à partir de la base jusqu'à l'extrémité inférieure de la fenêtre, et une seconde partie dans la section transversale augmentant dans la direction du mouvement de l'air et est agencée au niveau de ladite fenêtre.

7. Dispositif selon une ou plusieurs des revendications précédentes, comprenant, à l'intérieur de la chambre étanche, une soupape à une voie qui est adaptée pour permettre le passage des pertes d'air qui proviennent de la chambre pour collecter les liquides exsudés vers la source d'aspiration, empêchant leur retour, ladite soupape comprenant un corps pour contenir un élément de contrôle d'écoulement et une base pour mettre en prise ledit corps, **caractérisé en ce que** ladite base est formée de manière monolithique avec la structure du dispositif.

8. Dispositif selon une ou plusieurs des revendications précédentes, comprenant, à l'intérieur de la chambre pour collecter les liquides exsudés, une soupape de pression négative maximum, qui comprend un élément de contrôle d'écoulement qui est poussé par un ressort calibré de manière appropriée afin de bloquer un orifice pour le raccordement à l'atmosphère, **caractérisé en ce que** ledit élément de contrôle d'écoulement est prévu avec un joint torique conçu pour venir en contact avec la paroi plate au niveau du bord dudit orifice.
